# FASCICULE DE BREVET EUROPEEN

(11) **EP 3 641 885 B1**
(45) Date de publication et mention de la délivrance du brevet: **03.07.2024**
(21) Numéro de dépôt: 18731842.3
(22) Date de dépôt: 19.06.2018
(51) Int. Cl.: A61N 7/00

(54) **PROCÉDÉ DE COMMANDE POUR LE TRAITEMENT D'UN TISSU CÉRÉBRAL**
STEUERUNGSVERFAHREN ZUR BEHANDLUNG VON GEHIRNGEWEBE
CONTROL METHOD FOR THE TREATMENT OF BRAIN TISSUE

(30) Priorité: 19.06.2017 FR 1755565
(43) Date de publication de la demande: 29.04.2020
(73) Titulaire: Assistance Publique Hôpitaux de Paris, 75004 Paris (FR); Sorbonne Université, 75006 Paris (FR); Carthera, 75013 Paris (FR)
(72) Inventeur: CARPENTIER, Alexandre, 75116 Paris (FR); BOUCHOUX, Guillaume, 69100 Villeurbanne (FR); CANNEY, Michael, Denver, CO 80203 (US); LACOSTE, François, 75014 Paris (FR)
(74) Mandataire: Be IP
(86) Numéro de dépôt international: PCT/EP2018/066205
(87) Numéro de publication internationale: WO 2018/234282

(56) Documents cités:
- WO-A1-89/07907
- WO-A1-2016/202955
- US-A1- 2010 217 160

## Description

### DOMAINE DE L'INVENTION

La présente invention concerne le domaine technique général des procédés de commande pour le traitement d'un tissu cérébral - humain ou animal - par ultrasons afin d'aider un praticien dans le traitement d'une pathologie.

### ARRIERE PLAN DE L'INVENTION

On connaît différentes techniques permettant de traiter un tissu cérébral. Notamment, une technique connue consiste à utiliser un ensemble composé :
- d'une fenêtre acoustique constituée dans un matériau acoustiquement transparent, et
- d'une sonde ultrasonore apte à générer des ondes ultrasonores.

La fenêtre acoustique est implantée au niveau d'une ouverture ménagée dans la boîte crânienne d'un patient. Cette fenêtre permet de faciliter la transmission des ondes ultrasonores (générées par la sonde) à travers la boîte crânienne du patient. En effet, les os de la boîte crânienne ont tendance à perturber la transmission des ondes ultrasonores.

Le principe de fonctionnement d'un tel ensemble dans le cas du traitement d'un tissu cérébral d'intérêt est le suivant.

Une fois la fenêtre acoustique implantée dans le crâne du patient, une succession de séances de traitement sont prodiguées au patient pour traiter une pathologie qui l'affecte.

A chaque nouvelle séance de traitement, la sonde est positionnée au-dessus de la fenêtre acoustique, puis elle est activée pour émettre des ondes ultrasonores en direction du tissu cérébral d'intérêt à traiter.

Un inconvénient d'un tel ensemble est qu'il est difficile pour le praticien de positionner avec précision la sonde au droit de la fenêtre acoustique. En effet, une fois implantée, la fenêtre est recouverte par la peau du crâne, de sorte que sa position précise n'est pas connue.

Si la sonde ultrasonore n'est pas positionnée précisément au droit de la fenêtre transparente, des ondes ultrasonores peuvent être émises vers l'os du patient. Ceci peut :
- engendrer des brûlures au niveau du crâne du patient, et/ou
- limiter l'action thérapeutique des ondes ultrasonores, le tissu cérébral ne recevant pas une dose suffisante d'ondes ultrasonores pour garantir une bonne efficacité du traitement.

Le document WO 89/07907 décrit un système à ultrasons de production de lésions dans un cerveau. Le système comprend :
- un appareil de fixation du crâne,
- un appareil de traduction des données de position, et
- un transducteur à ultrasons commandé par ordinateur.

Un but de la présente invention est de proposer un procédé de commande d'une sonde pour le traitement d'un tissu cérébral à partir d'un ensemble composé d'une fenêtre acoustique et de la sonde ultrasonore, et permettant de pallier au moins l'un des inconvénients précités.

Notamment, un but de l'invention est de proposer un procédé de commande d'une sonde pour le traitement d'un tissu cérébral à partir d'un ensemble composé d'une fenêtre acoustique et de la sonde ultrasonore permettant :
- de limiter les risques de brûlure d'un patient et/ou
- d'améliorer l'efficacité du traitement par ondes ultrasonores.

### BREVE DESCRIPTION DE L'INVENTION

A cet effet, l'invention propose un procédé de commande conforme à la revendication 1.

Des aspects préférés mais non limitatifs de la présente invention sont les suivants :
- la phase de détection peut comprendre en outre une étape de recentrage de la sonde au-dessus de la fenêtre acoustique,
- l'étape de recentrage peut comprendre une sous-étape consistant à comparer la position du barycentre des transducteurs de la sonde avec la position du barycentre des transducteurs de la sonde situés au-dessus de la fenêtre acoustique,
- la phase de détection peut comprendre en outre une étape d'orientation de la sonde en fonction de la profondeur du tissu cérébral à traiter,
- la phase de détection peut comprendre en outre une étape consistant à calculer des paramètres de traitement utilisés lors d'une phase de traitement du tissu cérébral,
- les paramètres de traitement peuvent comprendre l'intensité et la durée d'émission des ondes ultrasonores à générer par les transducteurs situés au-dessus de la fenêtre acoustique,
- l'étape consistant à calculer des paramètres de traitement peut comprendre au moins l'une des sous-étapes suivantes prise seule ou en combinaison :
   o le calcul d'au moins un paramètre de traitement en fonction du nombre de transducteurs situés au-dessus de la fenêtre acoustique,
   o le calcul d'au moins un paramètre de traitement en fonction d'un coefficient d'atténuation ou de réflexion associé à la fenêtre acoustique ou aux tissus situés entre la sonde et la fenêtre, et/ou d'un coefficient d'atténuation associé à la matière constituant un tissu cérébral traversé par les ondes ultrasonores,
- l'étape consistant à traiter chaque onde ultrasonore d'écho peut comprendre la comparaison de l'onde ultrasonore d'écho à des signaux de référence, et la détection d'une variation d'une intensité de l'onde ultrasonore d'écho et/ou une variation de son délai d'apparition, Cette étape peut aussi comporter une étape d'écoute de la cavitation, afin de surveiller l'effet des ultrasons sur les tissus, grâce à des capteurs acoustiques disposés dans la sonde et reliés à une électronique.
- la phase de détection peut comprendre une étape consistant à calculer le nombre de transducteurs situés au-dessus de la fenêtre acoustique,
- la phase de détection peut en outre comprendre les étapes consistant à :
   o comparer le nombre calculé de transducteurs situés au-dessus de la fenêtre acoustique à une valeur seuil,
   o si le nombre de transducteurs situés au-dessus de la fenêtre acoustique est supérieur à la valeur seuil, envoyer une première information au praticien, la première information consistant à lui demander d'immobiliser la sonde,
   o Si le nombre de transducteurs situés au-dessus de la fenêtre acoustique est inférieur à la valeur seuil, envoyer une deuxième information au praticien, la deuxième information consistant à lui demander de déplacer la sonde sur le crâne du patient,
- la phase de détection peut comprendre une étape consistant à émettre une information de déplacement, ladite information de déplacement indiquant au praticien une direction de déplacement de la sonde,

L'invention concerne également un produit programme d'ordinateur conforme à la revendication 12.

L'invention concerne également un dispositif de traitement conforme à la revendication 13.

### BREVE DESCRIPTION DES DESSINS

D'autres avantages et caractéristiques du procédé de commande d'une sonde pour le traitement d'un tissu cérébral ressortiront mieux de la description qui va suivre de plusieurs variantes d'exécution, données à titre d'exemples non limitatifs, à partir des dessins annexés sur lesquels :
- La figure 1 illustre schématiquement un ensemble de traitement par ultrasons comprenant une fenêtre acoustique et une sonde ultrasonore,
- La figure 2 illustre schématiquement les étapes d'un procédé de commande d'une sonde pour le traitement d'une pathologie à partir de l'ensemble de traitement par ultrasons,
- Les figures 3A à 3D illustrent schématiquement en vue de dessus différentes positions de la sonde ultrasonore relativement à la fenêtre acoustique.

### DESCRIPTION DETAILLEE DE L'INVENTION

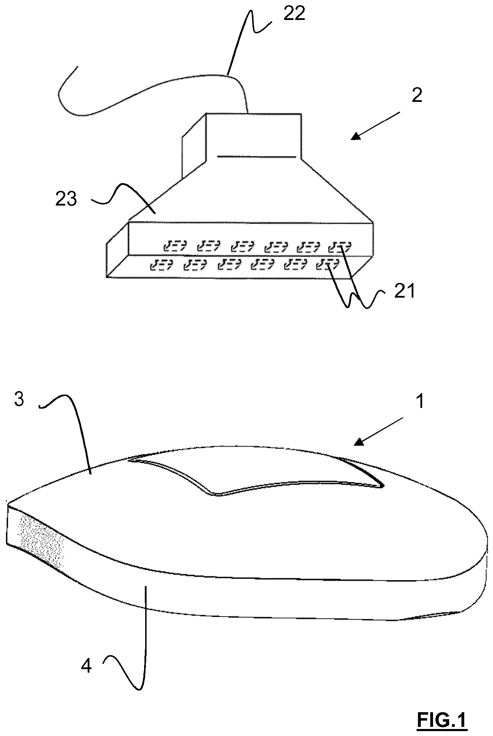

On va maintenant décrire plus en détails un exemple de procédé de commande d'une sonde pour le traitement d'un tissu cérébral à partir d'un ensemble de traitement (composé d'une fenêtre acoustique et d'une sonde ultrasonore) en référence aux figures 1 à 3. Dans ces différentes figures, les éléments équivalents sont désignés par la même référence numérique.

### 1. Principe général

L'ensemble pour le traitement d'un tissu cérébral comprend :
- Une fenêtre acoustique 1, et
- Une sonde 2 apte à générer des ondes ultrasonores.

Cet ensemble permet à un praticien de traiter le tissu cérébral en utilisant des ultrasons.

La fenêtre 1 est destinée à être implantée dans le patient, notamment au niveau d'une ouverture ménagée dans sa boîte crânienne 4. Ceci fournit une protection au cerveau et empêche sa déformation du fait des changements de pression. La fenêtre 1 est avantageusement constituée dans un matériau acoustiquement transparent - matériau polymère (tel que du polyéthylène, du polystyrène, de l'acrylique, du polyétheréthercétone (PEEK) ou du poly (méthacrylate de méthyle) (PMMA)) ou un élastomère thermoplastique (tel que du PEBAX) - pour permettre le passage des ondes ultrasonores générées par la sonde 2 afin de traiter un tissu cérébral.

La sonde 2 est adaptée pour être manipulée par le praticien, ou par un système de déplacement automatique portant la sonde. Elle comprend un boîtier 23 dans lequel sont logés des transducteurs 21 pour la génération d'ondes ultrasonores. Les transducteurs peuvent être arrangés en réseau linéaire ou matriciel de transducteurs à commande de phase (également connus sous le nom de « transducteurs phased array ». De tels transducteurs à commande de phase peuvent être commandés indépendamment pour générer des signaux acoustiques ayant des phases différentes afin de faire varier la direction de faire varier la direction de propagation des ondes ultrasonores. Le boitier est relié à un dispositif de commande par l'intermédiaire d'un câble électriquement conducteur 22.

La sonde 2 est destinée à être positionnée au droit de la fenêtre 10 pour permettre de traiter une zone cible. Toutefois, une fois implanté, il est difficile de déterminer la position de la fenêtre acoustique 1, celle-ci étant recouverte par la peau du crâne du patient. Il est aussi difficile de déterminer quelle sera la fraction des ondes acoustiques émises qui atteindra effectivement la cible après traversée de la peau du patient, en particulier si des cheveux sont présents, de la fenêtre acoustique et des tissus cérébraux proximaux.

C'est pourquoi les inventeurs ont développé un procédé de commande d'une sonde pour le traitement d'un tissu cérébral à partir d'un ensemble composé d'une fenêtre acoustique et de la sonde ultrasonore.

### 2. Procédé de commande

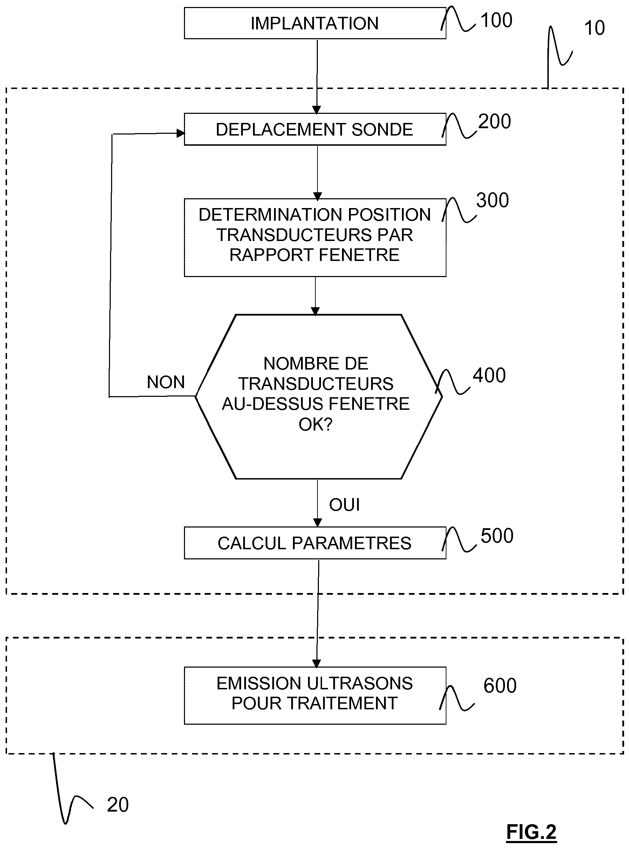

On va maintenant décrire un exemple de procédé de commande en référence à la figure 2.

Une fois la fenêtre implantée (étape 100), le procédé comprend deux phases :
- Une première phase de détection 10 consistant à assister le praticien dans le positionnement de la sonde ultrasonore 2 relativement à la fenêtre acoustique 1,
- Une deuxième phase de traitement 20 consistant à générer des ondes ultrasonores permettant de traiter un tissu cérébral.

Durant la phase de détection, le dispositif de commande contrôle la sonde ultrasonore 2 pour permettre la détection de la position des transducteurs 21 relativement à la fenêtre acoustique 1. Par exemple le dispositif de commande contrôle le basculement de la sonde 2 dans un mode de fonctionnement dit « *mode échographique A* » (aussi appelé « A-scan » ou « A-mode »). Le mode échographique A est basé sur l'émission d'une information acoustique et la réception d'échos le long d'une ligne de propagation. En variante, le dispositif de commande peut contrôler des émetteurs/récepteurs spécifiques intégrés à la sonde 2 et associés aux différents transducteurs 21.

Durant la phase de traitement, le dispositif de commande contrôle la sonde ultrasonore 2 pour permettre le traitement d'un tissu cérébral d'intérêt. Par exemple le dispositif de commande contrôle le basculement de la sonde 2 dans un mode de fonctionnement dit *« de traitement »* durant lequel chaque transducteur 21 activé émet des ondes ultrasonores thérapeutiques (focalisées ou non focalisées) de plus forte intensité.

### 2.1. Phase de détection

La phase de détection permet de déterminer si les transducteurs de la sonde sont positionnés :
- au-dessus de la fenêtre acoustique 1 ou
- au-dessus de la boîte crânienne 4 du patient.

Cette phase de détection permet également de déterminer si le couplage acoustique entre la sonde et le tissu cible est acceptable, notamment pour les transducteurs détectés comme étant situés au-dessus de la fenêtre acoustique.

Pour déterminer si les transducteurs de la sonde sont correctement positionnés d'une part, et évaluer la qualité du couplage acoustique d'autre part, une solution peut être d'utiliser la technique dite de *« réflexion échographique »*

Cette technique de *« réflexion échographique »* peut être basée sur l'évaluation de l'énergie contenue dans l'écho de retour. Plus précisément, si pour un transducteur considéré :
- l'énergie contenue dans le signal écho de retour est très élevée (i.e. supérieure à une première valeur seuil d'énergie dite *« maximum »*)*,* alors le transducteur considéré est au droit de l'os de la boîte crânienne ;
- l'énergie contenue dans le signal écho de retour est très faible (i.e. inférieure à une deuxième valeur seuil d'énergie dite *« minimum »*)*,* alors le transducteur considéré s'étend au droit de la fenêtre acoustique ;
- l'énergie contenue dans le signal écho de retour est comprise entre la valeur seuil d'énergie minimum et la valeur seuil d'énergie maximum, alors le transducteur s'étend au droit de la fenêtre, mais le couplage acoustique est de mauvaise qualité, par exemple en raison de bulles d'air autour des cheveux du patient.

En variante (ou en combinaison), la technique de *« réflexion échographique »* peut être basée sur l'évaluation d'un temps de vol de l'écho de retour. Plus précisément si pour un transducteur considéré :
- la durée entre l'émission d'une information acoustique et la réception d'un écho de retour est inférieure à une première valeur seuil temporelle, alors le couplage acoustique est de mauvaise qualité, par exemple en raison de bulles d'air autour des cheveux du patient ;
- la durée entre l'émission d'une information acoustique et la réception d'un écho de retour est inférieure à une deuxième valeur seuil temporelle supérieure à la première valeur seuil temporelle, alors le couplage acoustique est de bonne qualité mais le transducteur considéré est au droit de l'os de la boîte crânienne ;
- la durée entre l'émission d'une information acoustique et la réception d'un écho de retour est supérieure à la deuxième valeur seuil temporelle (ou à une troisième valeur seuil temporelle supérieure aux première et deuxième valeurs seuil temporelle), alors le couplage acoustique est de bonne qualité et le transducteur considéré est au droit de la fenêtre acoustique.

La phase de détection 10 comprend une étape de déplacement 200 de la sonde ultrasonore 2. Le boîtier 23 de la sonde 2 est plaqué sur la peau 3 de la boîte crânienne 4 du patient, puis déplacé. Lors du déplacement du boitier 23, le dispositif de commande émet des signaux de contrôle aux transducteurs 21 (ou aux émetteurs/récepteurs spécifiques) pour déterminer leur position relativement à la fenêtre acoustique 1. Ces signaux de contrôle peuvent être émis périodiquement (toute les une milliseconde ou toute les une seconde en fonction de la vitesse de déplacement de la sonde).

A chaque réception d'un signal de contrôle, chaque transducteur 21 (ou émetteur/récepteur spécifique) génère des ondes ultrasonores de faible intensité vers le crâne 4 du patient, et détecte des échos émis par réflexion.

Les échos enregistrés par chaque transducteur 21 (ou émetteur/récepteur spécifique) sont transmis au dispositif de commande par l'intermédiaire du câble 22.

Pour chaque transducteur 21, le dispositif de commande traite les échos enregistrés afin de déterminer (étape 300) si le transducteur (ou l'émetteur/récepteur spécifique) est positionné au-dessus :
- de la fenêtre acoustique 1, ou au-dessus,
- de la boîte crânienne 4 du patient.

Cette détermination de la position de chaque transducteur 21 (ou émetteur/récepteur spécifique) peut être obtenue en comparant chaque écho enregistré à des signaux de référence et/ou en détectant une variation du niveau d'écho et/ou de son délai d'apparition (par exemple une diminution de son intensité ou un retard de son apparition).

A chaque émission d'un signal de contrôle, le dispositif de commande est apte à déterminer si un transducteur est situé au-dessus de la fenêtre acoustique 1 ou non, et donc à calculer le nombre de transducteurs 21b situés au-dessus de la fenêtre acoustique 1.

Ce nombre de transducteurs 21b est comparé (étape 400) à une valeur seuil pour déterminer si suffisamment de transducteurs sont positionnés au-dessus de la fenêtre acoustique 1 pour permettre le traitement du tissu cérébral. Bien entendu, la valeur seuil peut varier en fonction notamment du traitement souhaité.

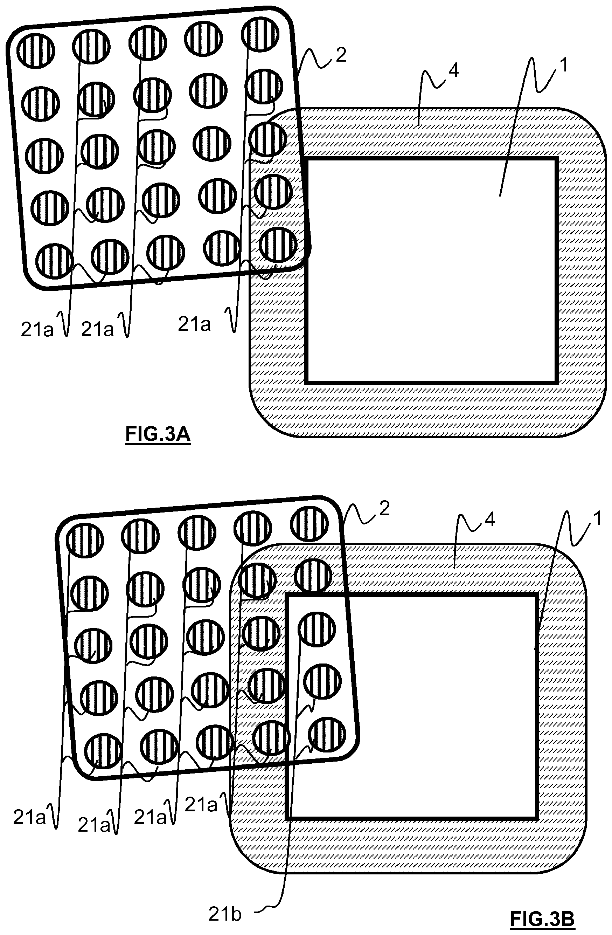

Si le nombre de transducteurs 21b situés au-dessus de la fenêtre acoustique 1 est inférieur à la valeur seuil, alors trop peu de transducteurs sont correctement positionnés pour assurer une bonne efficacité du traitement. Par exemple en référence à la figure 3A, on a illustré un exemple de positionnement de la sonde 2 relativement à la fenêtre acoustique 1 dans lequel aucun transducteur n'est positionné au-dessus de la fenêtre acoustique 1. En référence à la figure 3B, on a illustré un exemple de positionnement de la sonde 2 dans lequel seul trois transducteurs 21b s'étendent au-dessus de la fenêtre acoustique 1, la valeur seuil étant par exemple de « 9 ».

Dans ces deux cas, le nombre de transducteurs 21b situés au droit de la fenêtre acoustique 1 est insuffisant. Les étapes de déplacement du boitier 23 et de détermination de la position des transducteurs 21 sont réitérées jusqu'à obtention d'un nombre suffisant de transducteurs 21b positionnés sur la fenêtre acoustique 1.

Avantageusement, le procédé peut comprendre une étape d'émission d'une information au praticien (ou au système de déplacement automatique portant la sonde) pour lui indiquer une direction dans laquelle déplacer la sonde 2. Cette information peut consister en un stimulus visuel (affiché sur des moyens d'affichage du dispositif de commande) et/ou sonore (émis sur un hautparleur du dispositif de commande) et/ou tactile (par mise en vibration du boitier 23 de la sonde 2).

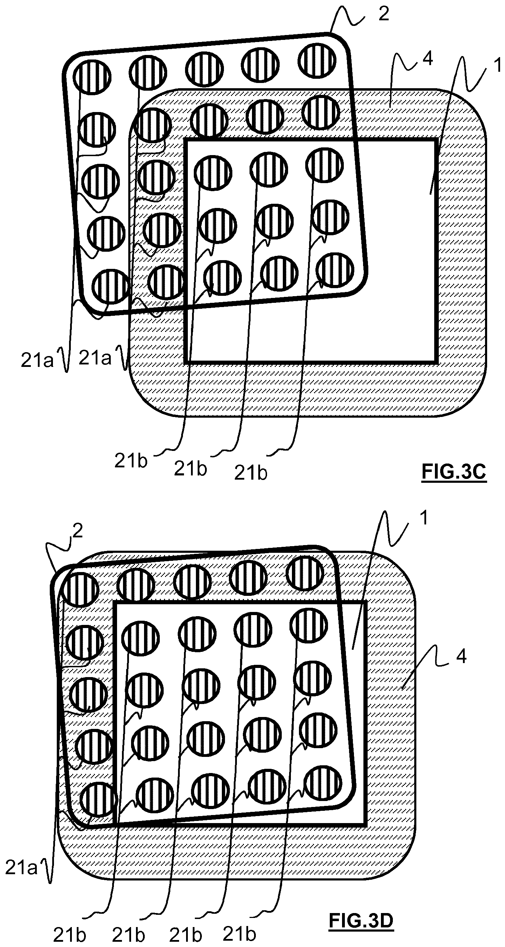

Si le nombre de transducteurs 21b situés au-dessus de la fenêtre acoustique 1 est supérieur à la valeur seuil, alors suffisamment de transducteurs sont correctement positionnés pour assurer une bonne efficacité du traitement. Par exemple en référence à la figure 3C, on a illustré un exemple de positionnement de la sonde 2 relativement à la fenêtre acoustique 1 dans lequel neuf transducteurs 21b sont positionnés au-dessus de la fenêtre acoustique 1, la valeur seuil étant par exemple de « 9 ». En référence à la figure 3D, on a illustré un exemple de positionnement de la sonde dans lequel douze transducteurs 21b s'étendent au-dessus de la fenêtre acoustique 1, la valeur seuil étant égale à « 9 ».

Dans ces deux cas, le nombre de transducteurs 21b positionnés correctement étant suffisant, les étapes suivantes du procédé peuvent être mise en oeuvre.

Dans une variante de réalisation, le procédé peut comprendre une étape de recentrage de la sonde au-dessus de la fenêtre acoustique, même si le nombre de transducteurs correctement positionné est suffisant. Cette étape permet d'assurer une répétabilité du traitement entre deux séances de traitement successives. L'étape de recentrage comprend par exemple les sous-étapes consistant à :
- comparer la position du barycentre de l'ensemble des transducteurs de la sonde avec la position du barycentre des transducteurs de la sonde identifiés comme étant au-dessus de la fenêtre acoustique,
- commander le déplacement de la sonde si la distance entre le barycentre de l'ensemble des transducteurs de la sonde et le barycentre des transducteurs de la sonde correctement positionnés au-dessus de la fenêtre acoustique est supérieure à une valeur seuil prédéfinie.

Avantageusement, le dispositif de commande peut émettre un stimulus visuel et/ou sonore pour avertir le praticien qu'il n'est plus nécessaire de déplacer la sonde 2. Le praticien maintien alors la sonde 2 en position.

Une fois la sonde correctement positionnée, le procédé peut comprendre une étape d'orientation de la sonde pour émettre les ondes ultrasonores dans une direction d'intérêt. Cette direction d'intérêt dépend notamment de la taille de la fenêtre acoustique et/ou de la profondeur du tissu cérébral à traiter. En effet, les dimensions de la fenêtre acoustique peuvent varier en fonction de la profondeur du tissu cérébral (notamment une tumeur) à traiter ou imager, une fenêtre de petites dimensions étant préférable. Par exemple, dans le cas d'une tumeur profonde, les dimensions de la fenêtre acoustique peuvent être inférieure aux dimensions de la tumeur, alors que dans le cas d'une tumeur superficielle (i.e. proche de la boîte crânienne), les dimensions de la fenêtre seront de préférence égales (ou supérieures) aux dimensions de la tumeur. En effet, dans le cas d'une tumeur profonde, il est possible de traiter toute la tumeur à partir d'une fenêtre acoustique (de plus petites dimensions que la tumeur) en faisant varier l'orientation de la sonde.

Ainsi le procédé peut comprendre une étape d'orientation de la sonde pour émettre les ondes ultrasonores dans une (ou plusieurs) direction(s) d'intérêt afin de traiter le tissu cérébral cible en émettant des ondes ultrasonores selon des angles d'incidence différents.

Le dispositif de commande calcule 500 ensuite des paramètres de traitement comprenant notamment l'intensité et la durée d'émission des ondes ultrasonores à générer par les transducteurs 21b situés au-dessus de la fenêtre acoustique 1. Egalement dans le cas où la sonde comprend des transducteurs « à commande de phase » multidirectionnels, les paramètres de traitement peuvent comprendre la direction d'émission des ondes ultrasonores.

Ces paramètres de traitement sont calculés en fonction du nombre de transducteurs 21b situés au-dessus de la fenêtre acoustique 1.

Par exemple dans la configuration illustrée à la figure 3D où douze transducteurs 21b sont situés au-dessus de la fenêtre acoustique 1, l'intensité calculée et/ou la durée d'émission calculée seront éventuellement inférieures à l'intensité calculée et/ou à la durée calculée dans la configuration illustrée à la figure 3C où seulement neuf transducteurs 21b sont positionnés au-dessus de la fenêtre acoustique 1.

### 2.2. Phase de traitement

Un fois les paramètres de traitement calculés, le dispositif de commande fait basculer la sonde 2 dans le mode de fonctionnement efficace.

Les transducteurs 21b situés au-dessus de la fenêtre acoustique 1 sont activés tandis que les transducteurs 21a situés au-dessus de la boîte crânienne 4 sont désactivés.

En réponse à un signal d'entrée (i.e. un signal de génération d'ultrasons), les transducteurs 21b activés peuvent émettre des ondes ultrasonores qui traversent la fenêtre acoustique 1 et se propagent jusqu'au tissu cérébral à traiter. Les transducteurs 21a désactivés ne peuvent quant à eux pas émettre d'ondes ultrasonores en réponse à un signal d'entrée.

Ceci permet d'éviter les risques de brûlure du patient.

Durant la phase de traitement, plusieurs générations successives d'ondes ultrasonores par les transducteurs 21b activés peuvent être mises en oeuvre successivement. Avantageusement, une étape de repérage de la position et de l'orientation de la sonde peut être effectuée entre chaque génération d'ondes ultrasonores par les transducteurs 21b activés. Ceci permet de garantir l'efficacité du traitement à chaque génération successive d'ondes ultrasonores par les transducteurs 21b activés.

On va maintenant présenter deux exemples de traitement réalisés à partir du procédé décrit précédemment.

### 2.3. Premier Exemple de traitement

Un premier exemple de traitement comprend les étapes suivantes.

Une fenêtre acoustique est implantée pendant la résection chirurgicale effectuée pour l'ablation de la tumeur. Ainsi, une chirurgie distincte n'est pas nécessaire ultérieurement pour l'implantation de la fenêtre acoustique.

Une succession de séances de traitement peuvent ensuite être prodiguées au patient pour traiter la pathologie qui l'affecte.

Avant chaque nouvelle séance de traitement, le praticien effectue une IRM et utilise un outil logiciel pour mettre en évidence la tumeur et la région d'amélioration / infiltration sur les images T2.

Un traitement est simulé avec un outil logiciel pour planifier la sonication, la mise en phase appropriée et le mouvement mécanique de la sonde pendant la sonication.

La température à la surface de la fenêtre, la température de points voisins à la surface du crâne ainsi que la qualité du couplage acoustique peuvent être surveillés, par exemple en mesurant la puissance acoustique réfléchie vers la sonde. Ceci permet d'arrêter le traitement si la température de la fenêtre et/ou de la surface du crâne du patient dépasse une valeur seuil critique.

Des microbulles d'agents de contraste peuvent être injectées au patient pour améliorer l'efficacité du traitement par ondes ultrasonores. En effet, les oscillations de microbulles d'agents de contraste ultrasonore sous l'action d'ultrasons engendrent une modulation de la perméabilité des barrières biologiques des cellules cancéreuses.

Les ondes ultrasonores sont dirigées vers le tissu cérébral à traiter. La cavitation des microbulles est surveillée. Chaque point du tissu cérébral à traiter est soumis à des ondes ultrasonore selon une "dose de cavitation" donnée, ou à une quantité maximale d'impulsions (e.x. 100 impulsions, etc.).

À chaque point de sonication, le système compense l'atténuation ou la réflexion des ondes ultrasonores due à la fenêtre acoustique, aux tissus cérébraux, et à l'éventuelle présence d'un vide de la tumeur après la résection. La qualité du couplage et du positionnement de la sonde peuvent être contrôlés à l'aide de marqueurs de positionnement.

Avantageusement, l'étape 500 de calcul des paramètres de traitement peut tenir compte de la durée de vie des microbulles d'agent de contraste (i.e. différence de temps entre l'injection des microbulles dans l'organisme et leur élimination au niveau des capillaires pulmonaires) dans la détermination d'une durée pour la séance de traitement.

Avantageusement, l'étape 500 de calcul des paramètres de traitement peut aussi tenir compte des signaux dits de cavitation enregistrés par un capteur ultrasonore disposé dans la sonde afin de recevoir à travers la fenêtre les émissions ultrasonores émises au sein du tissu.

### 2.4. Deuxième exemple de traitement

Un deuxième exemple de traitement peut comprendre les étapes suivantes.

Après implantation de la fenêtre acoustique, une imagerie par résonance magnétique (prétraitement IRM) peut être mise en oeuvre pour segmenter :
- la zone tumorale par rapport à la fenêtre acoustique (par exemple dans le cas où des marqueurs IRM ont été incorporés dans la fenêtre acoustique), et
- la fenêtre acoustique par rapport à des marqueurs anatomiques extracorporels (par exemple oreilles, yeux, etc.) ou implantés dans l'os ou la peau.

Pour le traitement par ondes ultrasonores, le praticien peut par exemple pointer ces marqueurs extracorporels avec la sonde si celle-ci est associée à un système de neuro-navigation. Un tel système permet de guider le praticien dans le positionnement et l'orientation de la sonde par rapport à la fenêtre acoustique et par rapport à la position du tissu cérébrale (tumeur) à traiter. L'association de la sonde ultrasonore à un système de neuro-navigation permet d'améliorer la précision spatiale dans la détermination de la position et de l'orientation de la sonde.

Le positionnement de la sonde et son couplage avec la fenêtre acoustique peuvent ensuite être vérifiés périodiquement (y compris lors du traitement par ultrasons, pour détecter d'éventuels mouvement du patient etc.) en utilisant une "signature acoustique" spécifique à partir de la fenêtre acoustique.

Le lecteur aura compris que de nombreuses modifications peuvent être apportées à l'invention décrite précédemment sans sortir matériellement des nouveaux enseignements et des avantages décrits ici.

Par conséquent, toutes les modifications de ce type sont destinées à être incorporées à l'intérieur de la portée des revendications jointes.

## Revendications

1. Procédé de commande d'une sonde (2) pour le traitement d'un tissu cérébral à partir d'un ensemble comprenant la sonde (2) et une fenêtre acoustique (1) destinée à être implantée au niveau d'une ouverture ménagée dans la boîte crânienne (4) d'un patient, la sonde (2) incluant une pluralité de transducteurs (21) de génération d'ondes ultrasonores et étant destinée à être positionnée au droit de la fenêtre acoustique (1),
**caractérisé en ce que** le procédé comprend :
- une phase de détection (10) :
o des transducteurs (21a) de la sonde (2) situés au-dessus de la boîte crânienne (4) du patient, et
o des transducteurs (21b) de la sonde (2) situés au-dessus de la fenêtre acoustique (1),
pour permettre :
o la désactivation des transducteurs (21a) de la sonde (2) situés au-dessus de la boîte crânienne (4) du patient,
o l'activation des transducteurs (21b) de la sonde (2) situés au-dessus de la fenêtre acoustique (1), lesdits transducteurs étant aptes à générer des ondes ultrasonores de traitement afin de traiter le tissu cérébral,
la phase de détection (10) comprenant les étapes consistant à :
- pour chaque transducteur (21), émettre une onde ultrasonore de mesure vers le crâne du patient à partir de la sonde,
- pour chaque transducteur (21), mesurer une onde ultrasonore d'écho réfléchie à partir de la sonde,
- traiter (300) chaque onde ultrasonore d'écho pour détecter si le transducteur associé est situé au-dessus de la fenêtre acoustique (1) ou au-dessus de la boîte crânienne (4),
- traiter chaque onde ultrasonore pour estimer une qualité de couplage acoustique entre la sonde et le tissu cible.

2. Procédé de commande selon la revendication 1, dans lequel la phase de détection comprend en outre une étape de recentrage de la sonde au-dessus de la fenêtre acoustique.

3. Procédé de commande selon la revendication 2, dans lequel l'étape de recentrage comprend une sous-étape consistant à comparer la position du barycentre des transducteurs de la sonde avec la position du barycentre des transducteurs (21b) de la sonde situés au-dessus de la fenêtre acoustique.

4. Procédé de commande selon l'une quelconque des revendications 1 à 3, dans lequel la phase de détection comprend en outre une étape d'orientation de la sonde en fonction de la profondeur du tissu cérébral à traiter.

5. Procédé de commande selon l'une quelconque des revendications 1 à 4, dans lequel la phase de détection comprend en outre une étape consistant à calculer (500) des paramètres de traitement utilisés lors d'une phase de traitement du tissu cérébral.

6. Procédé de commande selon la revendication 5, dans lequel les paramètres de traitement comprennent l'intensité et la durée d'émission des ondes ultrasonores à générer par les transducteurs (21b) situés au-dessus de la fenêtre acoustique (1).

7. Procédé de commande selon l'une quelconque des revendications 5 ou 6, dans lequel l'étape consistant à calculer des paramètres de traitement comprend au moins l'une des sous-étapes suivantes prise seule ou en combinaison :
- le calcul d'au moins un paramètre de traitement en fonction du nombre de transducteurs (21b) situés au-dessus de la fenêtre acoustique (1),
- le calcul d'au moins un paramètre de traitement en fonction d'un coefficient d'atténuation ou de réflexion associé à la fenêtre acoustique ou aux tissus situés entre la sonde et la fenêtre, et/ou d'un coefficient d'atténuation associé à la matière constituant un tissu cérébral traversé par les ondes ultrasonores.

8. Procédé de commande selon la revendication 1, dans lequel l'étape consistant à traiter (300) chaque onde ultrasonore d'écho comprend la comparaison de l'onde ultrasonore d'écho à des signaux de référence, et la détection d'une variation d'une intensité de l'onde ultrasonore d'écho et/ou une variation de son délai d'apparition.

9. Procédé de commande selon l'une quelconque des revendications 1 à 8, dans lequel la phase de détection (10) comprend une étape consistant à calculer le nombre de transducteurs (21b) situés au-dessus de la fenêtre acoustique (1).

10. Procédé de commande selon la revendication 9, dans lequel la phase de détection comprend en outre les étapes consistant à :
- Comparer (400) le nombre calculé de transducteurs (21b) situés au-dessus de la fenêtre acoustique (1) à une valeur seuil,
- Si le nombre de transducteurs (21b) situés au-dessus de la fenêtre acoustique (1) est supérieur à la valeur seuil, envoyer une première information au praticien, la première information consistant à lui demander d'immobiliser la sonde (2),
- Si le nombre de transducteurs (21b) situés au-dessus de la fenêtre acoustique (1) est inférieur à la valeur seuil, envoyer une deuxième information au praticien, la deuxième information consistant à lui demander de déplacer la sonde sur le crâne du patient.

11. Procédé de commande selon l'une quelconque des revendications 1 à 10, dans lequel la phase de détection (10) comprend une étape consistant à émettre une information de déplacement, ladite information de déplacement indiquant au praticien une direction de déplacement de la sonde (2).

12. Produit programme d'ordinateur comprenant des instructions de code de programmation destinées à exécuter les étapes du procédé selon l'une quelconque des revendications 1 à 11 lorsque ledit programme est exécuté sur un ordinateur connecté à une sonde (2) incluant une pluralité de transducteurs (21) de génération d'ondes ultrasonores.

13. Dispositif de traitement d'un tissu cérébral à partir d'un ensemble comprenant une fenêtre acoustique (1) destinée à être implantée au niveau d'une ouverture ménagée dans la boîte crânienne (4) d'un patient, et une sonde (2) incluant une pluralité de transducteurs (21) de génération d'ondes ultrasonores, la sonde étant destinée à être positionnée au droit de la fenêtre acoustique (1),
**caractérisé en ce que** le dispositif comprend des moyens reliés à la pluralité de transducteurs (21) pour la mise en oeuvre du procédé selon l'une des revendications 1 à 11.

## Patentansprüche

1. Verfahren zur Steuerung einer Sonde (2) zur Behandlung eines Hirngewebes in einer Anordnung, die die Sonde (2) und ein akustisches Fenster (1) umfasst, das bestimmt ist, im Bereich einer im Schädel (4) eines Patienten ausgebildeten Öffnung implantiert zu sein, wobei die Sonde (2) eine Vielzahl von Wandlern (21) zur Erzeugung von Ultraschallwellen enthält und bestimmt ist, senkrecht zu dem akustischen Fenster (1) positioniert zu sein,
**dadurch gekennzeichnet, dass** das Verfahren umfasst:
- eine Detektionsphase (10):
o der Wandler (21a) der Sonde (2), die sich oberhalb des Schädels (4) des Patienten befinden, und
o Wandlern (21b) der Sonde (2), die sich oberhalb des akustischen Fensters (1) befinden,
um zu gestatten:
o die Deaktivierung der Wandler (21a) der Sonde (2), die sich oberhalb des Schädels (4) des Patienten befinden,
o die Aktivierung der Wandler (21b) der Sonde (2), die sich oberhalb des akustischen Fensters (1) befinden, wobei die Wandler imstande sind, Ultraschallwellen zur Behandlung zu erzeugen, um das Hirngewebe zu behandeln,
wobei die Detektionsphase (10) die folgenden Schritte umfasst:
- für jeden Wandler (21), Senden einer Mess-Ultraschallwelle in Richtung des Schädels des Patienten, von der Sonde ausgehend,
- für jeden Wandler (21), Messen einer reflektierten Echo-Ultraschallwelle, von der Sonde ausgehend,
- Verarbeiten (300) jeder Echo-Ultraschallwelle, um zu ermitteln, ob sich der zugeordnete Wandler oberhalb des akustischen Fensters (1) oder oberhalb des Schädels (4) befindet,
- Verarbeiten jeder Ultraschallwelle, um eine akustische Kopplungsqualität zwischen der Sonde und dem Zielgewebe abzuschätzen.

2. Steuerungsverfahren nach Anspruch 1, wobei die Detektionsphase ferner einen Schritt der Rezentrierung der Sonde oberhalb des akustischen Fensters umfasst.

3. Steuerungsverfahren nach Anspruch 2, wobei der Schritt der Rezentrierung einen Unterschritt umfasst, der darin besteht, die Position des Schwerpunkts der Wandler der Sonde mit der Position des Schwerpunkts der Wandler (21b) der Sonde zu vergleichen, die sich oberhalb des akustischen Fensters befinden.

4. Steuerungsverfahren nach einem der Ansprüche 1 bis 3, wobei die Detektionsphase ferner einen Schritt der Ausrichtung der Sonde in Abhängigkeit von der Tiefe des zu behandelnden Hirngewebes umfasst.

5. Steuerungsverfahren nach einem der Ansprüche 1 bis 4, wobei die Detektionsphase ferner einen Schritt umfasst, der darin besteht, Behandlungsparameter zu berechnen (500), die bei einer Behandlungsphase des Hirngewebes verwendet werden.

6. Steuerungsverfahren nach Anspruch 5, wobei die Behandlungsparameter die Stärke und die Dauer des Sendens der Ultraschallwellen umfassen, die von den Wandlern (21b) zu erzeugen sind, die sich oberhalb des akustischen Fensters (1) befinden.

7. Steuerungsverfahren nach einem der Ansprüche 5 oder 6, wobei der Schritt, der darin besteht, die Behandlungsparameter zu berechnen, mindestens einen der folgenden Unterschritte allein oder in Kombination umfasst:
- das Berechnen von mindestens einem Behandlungsparameter in Abhängigkeit von der Anzahl der Wandler (21b), die sich oberhalb des akustischen Fensters (1) befinden,
- das Berechnen von mindestens einem Behandlungsparameter in Abhängigkeit von einem Dämpfungs- oder Reflexionskoeffizienten, der dem akustischen Fenster oder den Geweben zugeordnet ist, die sich zwischen der Sonde und dem Fenster befinden, und/oder von einem Dämpfungskoeffizienten, der der Materie zugeordnet ist, die ein Hirngewebe bildet, das von den Ultraschallwellen durchquert wird.

8. Steuerungsverfahren nach Anspruch 1, wobei der Schritt, der darin besteht zu behandeln (300), jede Echo-Ultraschallsonde den Vergleich der Echo-Ultraschallsonde mit Referenzsignalen und die Ermittlung einer Schwankung einer Stärke der Echo-Ultraschallsonde und/oder einer Schwankung der Zeit ihres Auftretens umfasst.

9. Steuerungsverfahren nach einem der Ansprüche 1 bis 8, wobei die Detektionsphase (10) einen Schritt umfasst, der darin besteht, die Anzahl der Wandler (21b) zu berechnen, die sich oberhalb des akustischen Fensters (1) befinden.

10. Steuerungsverfahren nach Anspruch 9, wobei die Detektionsphase ferner die folgenden Schritte umfasst:
- Vergleichen (400) der berechneten Anzahl der Wandler (21b), die sich oberhalb des akustischen Fensters (1) befinden, mit einem Grenzwert,
- wenn die Anzahl der Wandler (21b), die sich oberhalb des akustischen Fensters (1) befinden, größer als der Grenzwert ist, Senden einer ersten Information an den Arzt, wobei die erste Information darin besteht, ihn zu bitten, die Sonde (2) zu arretieren,
- wenn die Anzahl der Wandler (21b), die sich oberhalb des akustischen Fensters (1) befinden, kleiner als der Grenzwert ist, Senden einer zweiten Information an den Arzt, wobei die zweite Information darin besteht, ihn zu bitten, die Sonde auf dem Schädel des Patienten zu verlagern.

11. Steuerungsverfahren nach einem der Ansprüche 1 bis 10, wobei die Detektionsphase (10) einen Schritt umfasst, der darin besteht, eine Verlagerungsinformation zu senden, wobei die Verlagerungsinformation dem Arzt eine Verlagerungsrichtung der Sonde (2) vorgibt.

12. Rechnerprogrammprodukt, umfassend Programmcodebefehle, die dazu bestimmt sind, die Schritte des Verfahrens nach einem der Ansprüche 1 bis 11 auszuführen, wenn das Programm auf einem Rechner ausgeführt wird, der mit einer Sonde (2) verbunden ist, die eine Vielzahl von Wandlern (21) zur Erzeugung von Ultraschallwellen enthält.

13. Vorrichtung zur Behandlung eines Hirngewebes mit einer Anordnung, die ein akustisches Fenster (1) umfasst, das bestimmt ist, im Bereich einer in den Schädel (4) eines Patienten ausgebildeten Öffnung implantiert zu sein, und eine Sonde (2), die eine Vielzahl von Wandlern (21) zur Erzeugung von Ultraschallwellen enthält, wobei die Sonde bestimmt ist, senkrecht zu dem akustischen Fenster (1) positioniert zu sein,
**dadurch gekennzeichnet, dass** die Vorrichtung Mittel umfasst, die mit der Vielzahl von Wandlern (21) für die Durchführung des Verfahrens nach einem der Ansprüche 1 bis 11 verbunden sind.

## Claims

1. A method for controlling a probe (2) for the treatment of a brain tissue from an assembly comprising the probe (2) and an acoustic window (1) intended to be implanted at an opening arranged in the cranium (4) of a patient, the probe (2) including a plurality of ultrasonic wave generation transducers (21) and being intended to be positioned in line with the acoustic window (1),
**characterized in that** the method comprises:
- a detection phase (10) of:
o transducers (21a) of the probe (2) situated above the cranium (4) of the patient, and
o transducers (21b) of the probe (2) situated above the acoustic window (1),
to enable:
o the deactivation of the transducers (21a) of the probe (2) situated above the cranium (4) of the patient,
o the activation of the transducers (21b) of the probe (2) situated above the acoustic window (1), said transducers being able to generate ultrasonic treatment waves in order to treat the brain tissue,
the detection phase (10) comprising the steps of:
- emitting, for each transducer (21), an ultrasonic measurement wave toward the patient's skull from the probe,
- measuring, for each transducer (21), an ultrasonic echo wave reflected from the probe,
- processing (300) each ultrasonic echo wave to detect whether the associated transducer is situated above the acoustic window (1) or above the cranium (4),
- processing each ultrasonic wave to estimate an acoustic coupling quality between the probe and the target tissue.

2. The control method according to claim 1, wherein the detection phase further comprises a step of refocusing the probe above the acoustic window.

3. The control method according to claim 2, wherein the refocusing step comprises a sub-step of comparing the position of the barycentre of the probe transducers with the position of the barycentre of the probe transducers (21b) situated above the acoustic window.

4. The control method according to any one of claims 1 to 3, wherein the detection phase further comprises a step of orienting the probe depending on the depth of the brain tissue to be treated.

5. The control method according to any one of claims 1 to 4, wherein the detection phase further comprises a step of calculating (500) treatment parameters used during a brain tissue treatment phase.

6. The control method according to claim 5, wherein the treatment parameters comprise the intensity and duration of emission of the ultrasonic waves to be generated by the transducers (21b) situated above the acoustic window (1).

7. The control method according to any one of claims 5 or 6, wherein the step of calculating treatment parameters comprises at least one of the following sub-steps taken alone or in combination:
- calculating at least one treatment parameter as a function of the number of transducers (21b) situated above the acoustic window (1),
- calculating at least one treatment parameter as a function of an attenuation or reflection coefficient associated with the acoustic window or with the tissues situated between the probe and the window, and/or of an attenuation coefficient associated with the material constituting a brain tissue traversed by the ultrasonic waves.

8. The control method according to claim 1, wherein the step of processing (300) each ultrasonic echo wave comprises the comparison of the ultrasonic echo wave with reference signals, and the detection of a variation of an intensity of the ultrasonic echo wave and/or a variation in its appearance time.

9. The control method according to any one of claims 1 to 8, wherein the detection phase (10) comprises a step of calculating the number of transducers (21b) situated above the acoustic window (1).

10. The control method according to claim 9, wherein the detection phase further comprises the steps of:
- comparing (400) the calculated number of transducers (21b) situated above the acoustic window (1) with a threshold value,
- if the number of transducers (21b) situated above the acoustic window (1) is greater than the threshold value, sending first information to the practitioner, the first information consisting in asking him to immobilize the probe (2),
- if the number of transducers (21b) situated above the acoustic window (1) is smaller than the threshold value, sending second information to the practitioner, the second information consisting in asking him to displace the probe on the skull of the patient.

11. The control method according to any one of claims 1 to 10, wherein the detection phase (10) comprises a step of emitting displacement information, said displacement information indicating to the practitioner a direction of displacement of the probe (2).

12. A computer program product comprising programming code instructions intended to perform the steps of the method according to any one of claims 1 to 11 when said program is run on a computer connected to a probe (2) including a plurality of ultrasonic wave generation transducers (21).

13. A device for treating a brain tissue from an assembly comprising an acoustic window (1) intended to be implanted at an opening arranged in the cranium (4) of a patient, and a probe (2) including a plurality of ultrasonic waves generation transducers (21), the probe being intended to be positioned in line with the acoustic window (1),
**characterized in that** the device comprises means related to the plurality of transducers (21) for implementing the method according to any of claims 1 to 11.
